# EUROPÄISCHE PATENTSCHRIFT

(11) **EP 0 627 229 B1**
(45) Veröffentlichungstag und Bekanntmachung des Hinweises auf die Patenterteilung: **21.04.1999**
(21) Anmeldenummer: 94107827.1
(22) Anmeldetag: 20.05.1994
(51) Int. Cl.: A61M 5/315

(54) **Vorschubvorrichtung für eine Injektionsvorrichtung**
Dosing means for a syringe
Dispositif de dosage pour seringue

(30) Priorität: 04.06.1993 CH 1685/93
(43) Veröffentlichungstag der Anmeldung: 07.12.1994
(73) Patentinhaber: MEDIMPEX ETS., FL-9496 Balzers (LI)
(72) Erfinder: Huggenberger, Heinz, CH-3308 Grafenried (CH)
(74) Vertreter: Lusuardi, Werther Giovanni, Dr.

(56) Entgegenhaltungen:
- DE-A- 3 814 023
- US-A- 3 949 748

## Beschreibung

Die Erfindung bezieht sich auf Vorschubvorrichtung für eine Injektionsvorrichtung, gemäss dem Oberbegriff des Patentanspruchs 1.

Eine solche Vorrichtung mit einem Zahnstangenmechanismus ist bereits aus der EP-B1 0 037 696 bekannt. Nachteilig bei dieser bekannten Vorrichtung ist der Umstand, dass sich der Patient durch mehrfaches Drücken des Dosierknopfes eine Überdosis an Medikament verabreichen kann. Ein weiterer Nachteil besteht darin, dass die zu verabreichende Dosis in der Regel über das Vortreiben mehrerer Zähne erreicht wird, so dass durch eine Hin- und Herbewegung des Dosierknopfes in Richtung der Längsachse innerhalb des Dosishubes eine Überdosis erreicht werden kann.

Hier will die Erfindung Abhilfe schaffen. Der Erfindung liegt die Aufgabe zugrunde, eine Vorschubvorrichtung für eine Injektionsvorrichtung zu schaffen, die eine erhöhte Sicherheit bei einfachster Bedienung bietet.

Die Erfindung löst die gestellte Aufgabe mit einer Vorschubvorrichtung, welche die Merkmale des Anspruchs 1 aufweist, sowie einer Injektionsvorrichtung mit einer solchen Vorschubvorrichtung, welche die Merkmale des Anspruchs 7 aufweist.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellung eines Ausführungsbeispiels noch näher erläutert.

Es zeigen:
Fig. 1 einen Längsschnitt durch die erfindungsgemässe Vorschubvorrichtung; und
Fig. 2 einen Querschnitt längs der Linie A-A von Fig.1.

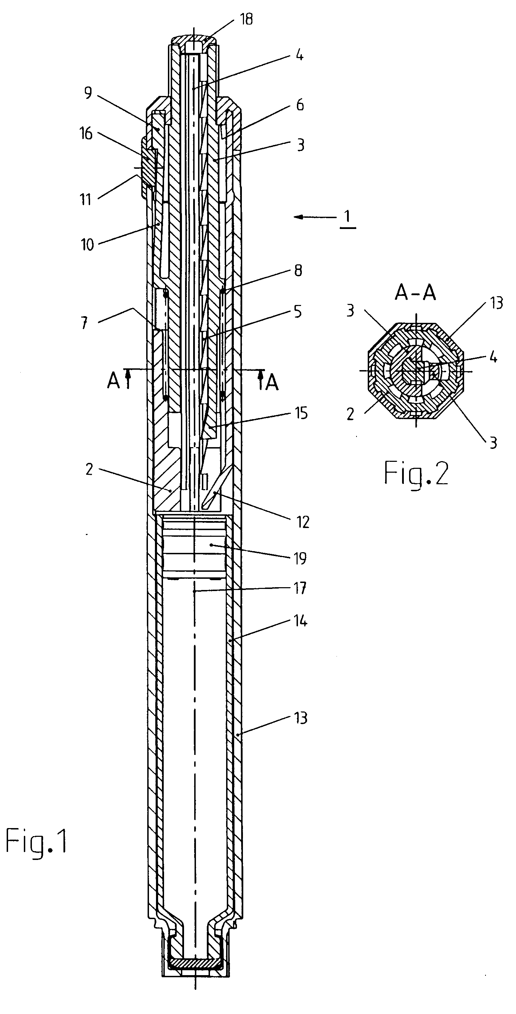

Die in den Fig. 1 und 2 dargestellte Vorschubvorrichtung 1 ist in eine aus einer Karpulenhülse 13 bestehenden Injektionsvorrichtung einsteckbar. Sie besteht im wesentlichen aus einer äusseren Hülse 2, einer aus der äusseren Hülse 2 nach hinten herausragenden, inneren Hülse 3 und einer gegen die Kraft einer Feder 8 mittels der inneren Hülse 3 vorschiebbaren Zahnstange 4. Die innere Hülse 3 weist an ihrem hinteren, aus der äusseren Hülse 2 herausragenden Ende einen Dosierknopf 18 auf. Die Zahnstange 4 ist mit einer Anzahl von gleichmässig langen Zähnen 5 versehen, deren Länge L einer vollen Injektionsdosis entsprechen. Die innere Hülse 3 ist zwischen zwei, in der äusseren Hülse 2 vorgesehenen Anschlägen 6 und 7 um den Betrag der Länge L vorwärts und rückwärts bewegbar.

Die innere Hülse 3 ist zudem mit Einrastmitteln versehen, welche aus einem an der inneren Hülse 3 in radialer Richtung federnd angebrachten Sicherungsschieber 10 mit einer Sperrklinke 9 bestehen. Die Sperrklinke 9 rastet bei Erreichen der vordersten Position der inneren Hülse 3 in eine in der Mantelfläche der Karpulenhülse 13 angebrachten Öffnung 11 ein. Die Vorschubvorrichtung 1 ist dadurch blockiert und kann erst nach Entsicherung der Sperrklinke 9 wieder betätigt werden.

Die innere Hülse 3 ist zudem mit einer Sperrklinke 15 versehen, welche in die Zahnstange 4 eingreift und diese beim Vorwärtsbewegen der inneren Hülse 3 nach vorne drückt. Für die Zahnstange 4 ist eine Rückstossicherung 12 vorgesehen, welche in Form einer an der äusseren Hülse 2 befestigten, gegen die Längsachse 17 der Vorschubvorrichtung 1 geneigten, in den Vorschubweg der Zahnstange 4 ragenden Klinke realisiert ist.

Die Öffnung 11 in der Karpulenhülse 13 ist durch einen lösbaren Sicherungsknopf 16 von aussen verschliessbar, wobei der Sicherungsknopf 16 die Sperrklinke 9 das Sicherungsschiebers 10 in der hintersten Position der inneren Hülse 3 fixiert, so dass die innere Hülse 3 gegenüber der äusseren Hülse 2 blockiert ist.

Die Vorschubvorrichtung 1 kann auf der Karpulenhülse 13, in deren vorderem Teil eine Karpule 14 einführbar ist einfach aufgesteckt werden und bildet dann eine vollständige Injektionsvorrichtung in Form eines Injektions-Pens.

Nachfolgend ist die Funktionsweise der Vorschubvorrichtung 1 näher beschrieben.
Nach Entfernung des Sicherungsknopfes 16 und Aufsetzen einer (nicht dargestellten) Nadel auf das vordere Ende der Karpulenhülse 13 kann durch Drücken des Dosierknopfes 18 das Medikament ausgeschüttet werden. Die Höhe der einzelnen Zähne 5 der Zahnstange 4 ist so gewählt, dass ein Zahn 5 genau einer Dosis entspricht. Der Benutzer drückt den Dosierknopf 18 bis der Sicherungsschieber 10 mit seiner Sperrklinke 9 in die Öffnung 11 der Karpulenhülse 13 einrastet; der Dosierknopf 18 bleibt dabei in der vorderen Position eingerastet. Innerhalb der Dosishöhe verursacht eine Hin- und Herbewegung des Dosierknopfes 18 keine Überdosis, da die Vorschubklinke 15 dabei innerhalb des gleichen Zahnes bleibt.

Gleichzeitig mit dem Einrasten der Sperrklinke 9 des Sicherungsschiebers 10 rastet auch die Rückstossicherung 12 in einen neuen Zahn 5. Damit ist garantiert, dass beim Entriegeln des Dosierknopfes 18 durch Hineindrücken des Sicherungsknopfes 16 die Zahnstange 4 beim Zurückgleiten des Dosierknopfes 18 nicht mit zurückgezogen wird. Die Vorschubklinke 15 rastet nun in einen neuen Zahn 5 ein.
Damit während des Transports und der Lagerhaltung nicht unbeabsichtigt der Karpulenstopfen 19 durch die Zahnstange 4 via Dosierknopf 18 bewegt wird, verhindert ein Sicherungsknopf 16 das Bewegen des Dosierknopfes 18.
Der Medikamentenbehälter, d.h. die Karpule 14 und die Karpulenhülse 13 (Pen aus Kunststoff) werden als funktionelle Einheit dem Benutzer abgegeben, d.h. nach der Entleerung der Karpule 14 kann der Kunststoff-Pen nicht mehr eingesetzt werden.

## Patentansprüche

1. Vorschubvorrichtung (1) für eine, eine Karpulenhülse (13) umfassende Injektionsvorrichtung, mit einer äusseren in die Karpulenhülse (13) eingeschobenen Hülse (2), einer aus der äusseren Hülse (2) nach hinten herausragenden, inneren Hülse (3) und einer gegen die Kraft einer Feder (8) mittels der inneren Hülse (3) vorschiebbaren Zahnstange (4), dadurch gekennzeichnet, dass
A) die Zahnstange (4) mit Zähnen (5) versehen ist, deren Länge L einer vollen Injektionsdosis entsprechen;
B) die innere Hülse (3) zwischen zwei, in der äusseren Hülse (2) vorgesehenen Anschlägen (6,7) um den Betrag der Länge L vor und zurück bewegbar ist;
C) die innere Hülse (3) mit Einrastmitteln (9,10) versehen ist, welche sie beim Erreichen der vordersten Position in einer Öffnung (11) der Karpulenhülse (13) gegenüber dieser in lösbarer Weise arretiert; und
D) eine Rückstosssicherung (12) für die Zahnstange (4) vorgesehen ist.

2. Vorschubvorrichtung (1) nach Anspruch 1, dadurch gekennzeichnet, dass die Einrastmittel (9;10) aus einem an der inneren Hülse (3) in radialer Richtung federnden angebrachten Sicherungsschieber (10) mit einer Sperrklinke (9) besteht, wobei die Sperrklinke (9) in eine in der Mantelfläche der Karpulenhülse (13) angebrachten Öffnung (11) bei Erreichen der vordersten Position der inneren Hülse (3) einrastet.

3. Vorschubvorrichtung (1) nach Anspruch 2, dadurch gekennzeichnet, dass die Öffnung (11) durch einen lösbaren Sicherungsknopf (16) von aussen verschliessbar ist, wobei der Sicherungsknopf (16) die Sperrklinke (9) in der hintersten Position der inneren Hülse (3) fixiert, so dass die innere Hülse (3) gegenüber der äusseren Hülse (2) blockiert ist.

4. Vorschubvorrichtung (1) nach einem der Ansprüche 1 bis 3, dadurch gekennzeichnet, dass die innere Hülse (3) mit einer Sperrklinke (15) versehen ist, welche in die Zahnstange (4) eingreift und diese beim Vorwärtsbewegen der inneren Hülse (3) nach vorne drückt.

5. Vorschubvorrichtung (1) nach einem der Ansprüche 1 bis 3, dadurch gekennzeichnet, dass die Rückstossicherung (12) in Form einer an der äusseren Hülse (2) befestigten, gegen die Längsachse (17) der Vorschubvorrichtung (1) geneigten, in den Vorschubweg der Zahnstange (4) ragenden Klinke ausgebildet ist.

6. Vorschubvorrichtung (1) nach einem der Ansprüche 1 bis 5, dadurch gekennzeichnet, dass die innere Hülse (3) an ihrem hinteren, aus der äusseren Hülse 2 herausragenden Ende einen Dosierknopf (18) aufweist.

7. Injektionsvorrichtung mit einer Vorschubvorrichtung (1) nach einem der Ansprüche 1 bis 6, gekennzeichnet durch eine Karpulenhülse (13), in deren vorderem Teil eine Karpule (14) einführbar ist und auf deren hinterem Teil die Vorschubvorrichtung (1) befestigbar ist.

## Claims

1. A feed mechanism (1) for an injection device comprising an ampoule casing (13) with an outer casing (2) slid into the ampoule casing (13), an inner casing (3) in said outer casing (2) and protruding rearwardly from said outer casing (2) and a toothed rack (4) forwardly moveable against the force of a spring means (8) by means of the inner casing (3),
**characterized in that**
A) the toothed rack (4) has a plurality of teeth (5), each tooth (5) having a length L corresponding to one full injection dose;
B) said inner casing (3) being movable forwardly and backwardly with the amount of the length L between two stop means (6;7) included within the outer casing (2);
C) latch means (9; 10) carried by said inner casing (3) for releasably engaging an aperture (11) within said ampoule casing (13) when said inner casing (3) reaches an extreme forward position to thereby prevent motion of said inner casing (3) relative to said ampoule casing (13); and
D) means (12) for preventing rearward motion of said toothed rack (4) are provided.

2. A feed mechanism (1) according to claim 1, characterized in that said latch means (9, 10) includes a slider arm (10) carried by said inner casing (3) and radially spring-urged, a pawl (9) carried by said slider arm (10), said pawl (9) being positioned to enter said aperture (11) lodged within the surface area of the ampoule casing (13) when said extreme forward position of the inner casing (3) is reached.

3. A feed mechanism (1) according to claim 2, characterized in that it includes a removable safety button (16) for closing said aperture (11) from the outside, said button (16) holding said pawl (9) in an extreme rearward position of said inner casing (3) such that the inner casing (3) is locked relative to said outer casing (2).

4. A feed mechanism (1) according to one of claims 1 through 3, characterized in that the inner casing (3) is fitted with a ratchet (15) engaging the toothed rack (4) which is forced forward when the inner casing (3) is being advanced.

5. A feed mechanism (1) according to one of claims 1 through 3, characterized in that the recoil safety means (12) is a pawl slanting relative to the longitudinal axis (17) of the said feed mechanism (1), entering the path of advance of the toothed rack (4) and affixed to the outer casing 2.

6. A feed mechanism (1) according to one of claims 1 through 5, characterized in that, at its rear end projecting from the outer casing (2), the inner casing (3) is fitted with a dosing button (18).

7. An injection device with a feed mechanism (1) according to one of the claims 1 to 6, characterized through a ampoule casing (13) for receiving an ampoule (14) of medicine at a forward end thereof and for receiving the feed mechanism (1) at a rearward end thereof.

## Revendications

1. Dispositif d'avancement (1) pour un dispositif d'injection comportant une douille (13) pour ampoule d'injection, présentant une douille (2) extérieure enfoncée dans la douille (13) pour ampoule d'injection, une douille intérieure (3) débordant vers l'arrière de la douille extérieure (2) et une crémaillère (4) qui peut être avancée au moyen de la douille intérieure (3) en opposition à la force d'un ressort (8), caractérisé en ce que
A) la crémaillère (4) est dotée de dents (5) dont la longueur L correspond à une dose complète d'injection;
B) la douille intérieure (3) peut être déplacée vers l'avant et vers l'arrière sur la longueur L entre deux butées (6, 7) prévues dans la douille extérieure (2);
C) la douille intérieure (3) est dotée de moyens d'encliquetage (9, 10) qui la bloquent de manière libérable par rapport à la douille (13) pour ampoule d'injection, dans une ouverture (11) de la douille pour ampoule d'injection, lorsque la douille intérieure a atteint la position située le plus en avant; et
D) il est prévu un verrouillage anti-recul (12) pour la crémaillère (4).

2. Dispositif d'avancement (1) selon la revendication 1, caractérisé en ce que les moyens d'encliquetage (9; 10) sont constitués d'un coulisseau de verrouillage (10), doté d'un cliquet de verrouillage (9), installé sur la douille intérieure (3) de manière élastique dans le sens radial, le cliquet de verrouillage (9) s'engageant dans une ouverture (11) pratiquée dans la surface d'enveloppe de la douille (13) pour ampoule d'injection lorsque la douille intérieure (3) a atteint sa position située le plus en avant.

3. Dispositif d'avancement (1) selon la revendication 2, caractérisé en ce que l'ouverture (11) peut être fermée par l'extérieur par l'intermédiaire d'un bouton de verrouillage (16) libérable, le bouton de verrouillage (16) immobilisant le cliquet de verrouillage (9) lorsque la douille intérieure (3) est située dans la position située le plus en arrière, de telle sorte que la douille intérieure (3) soit bloquée par rapport à la douille extérieure (2).

4. Dispositif d'avancement (1) selon l'une des revendications 1 à 3, caractérisé en ce que la douille intérieure (3) est dotée d'un cliquet de verrouillage (15) qui s'engage dans la crémaillère (4) et repousse cette dernière vers l'avant lorsque la douille intérieure (3) est avancée vers l'avant.

5. Dispositif d'avancement (1) selon l'une des revendications 1 à 3, caractérisé en ce que le verrouillage anti-recul (12) présente la forme d'un cliquet fixé sur la douille extérieure (2), incliné vers l'axe longitudinal (17) du dispositif d'avancement (1) et débordant dans le parcours d'avancement de la crémaillère (4).

6. Dispositif d'avancement (1) selon l'une des revendications 1 à 5, caractérisé en ce qu'à son extrémité arrière qui déborde de la douille extérieure (2), la douille intérieure (3) présente un bouton de dosage (18).

7. Dispositif d'injection doté d'un dispositif d'avancement (1) selon l'une des revendications 1 à 6, caractérisé par une douille (13) pour ampoule d'injection dans la partie avant de laquelle une ampoule d'injection (14) peut être insérée et sur la partie arrière de laquelle le dispositif d'avancement (1) peut être fixé.
